(19) Europäisches Patentamt
European Patent Office
Office européen des brevets

(11) Veröffentlichungsnummer: **0 367 974**
**A1**

(12) # EUROPÄISCHE PATENTANMELDUNG

(21) Anmeldenummer: **89118143.0**

(51) Int. Cl.[5]: **C07F 15/00, A61K 31/28**

(22) Anmeldetag: **30.09.89**

(30) Priorität: **07.10.88 DE 3834098**

(43) Veröffentlichungstag der Anmeldung: **16.05.90 Patentblatt 90/20**

(84) Benannte Vertragsstaaten: **AT BE CH DE ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Kolar, Cenek, Dr.**
**Deutschhausstrasse 20**
**D-3550 Marburg(DE)**
Erfinder: **Kraemer, Hans Peter, Dr.**
**Birkenweg 16**
**D-3550 Marburg(DE)**
Erfinder: **Dehmel, Konrad**
**Blumengarten 11**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Cis-Platin-Komplexe mit einem 1,3-Diaminopropan-Derivat als Liganden, Verfahren zu ihrer Herstellung und diese Verbindung enthaltende pharmazeutische Mittel.**

(57) Zytostatisch wirksame Cis-Platin-Derivate, die der nachfolgenden allgemeinen Formel I

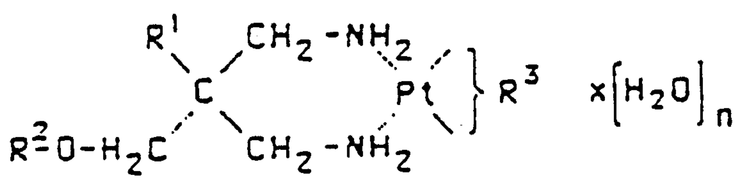

I

entsprechen,
worin
$R^1$ $C_1$-$C_6$-Alkyl oder $C_1$-$C_4$-Alkyloxymethyl
$R^2$ $C_1$-$C_4$-Alkyl oder $C_1$-$C_4$-Alkyloxyethyl
$R^1$ und $R^2$ zusammen eine Ketal-bildende Gruppe $-CH_2-O-CR^4(R^5)-$ mit
$R^4$ H oder $CH_3$ und
$R^5$ $CH_3$ oder Phenyl und
$R^3$ ein Anion der Cyclopropan-1,1-dicarbonsäure, Glycolsäure, alpha-$C_1$-$C_4$-Alkylglycolsäure, $C_1$-$C_4$-Alkyloxymalonsäure, Mesoxalsäure, Morpholin-4-yl-malonsäure oder Di-($C_1$-$C_4$-alkyl)-amino-malonsäure und
n 0, 1 oder 2 bedeuten,

EP 0 367 974 A1

Xerox Copy Centre

sowie Verfahren zu ihrer Herstellung und ihre Verwendung als Arzneimittel.

# Cis-Platin-Komplexe mit einem 1,3-Diaminopropan-Derivat als Liganden, Verfahren zu ihrer Herstellung und diese Verbindung enthaltende pharmazeutische Mittel

Die Erfindung betrifft neue Cis-Platin-Komplex-Verbindungen mit einem 1,3-Diaminopropan-Derivat und Malonsäure-oder alpha-Hydroxycarbonsäure-Derivaten als Liganden, Verfahren zu ihrer Herstellung und ein pharmazeutisches Mittel, in dem diese neuen Verbindungen enthalten sind.

Cis-Platin-Komplexe der allgemeinen Formel Cis-$L_2PtX_2$, wobei L ein neutraler Ligand wie $NH_3$ oder ein organisches Amin und X ein anionischer Ligand wie Chlorid oder ein Anion einer organischen Säure (Mono- und Oligocarbonsäuren) ist, besitzen antitumorale Wirksamkeit.

Cis-Platin-(II)-diamin-dichlorid und dessen Cyclobutan-1,1-dicarbonsäure-Derivat wurden als Arzneimittel eingeführt. In der EPA 0 232 784 A2 sind (Gem-heterocyclodimethanamin-N,N′)-platin-Komplexe beschrieben, die im anionischen Teil ein Dicarbonsäure-Derivat enthalten.

In EPA 0 174 542 sind solche Verbindungstypen sowie Alkyloxyalkyl-1,3-diaminopropan-platin-Komplex-Derivate beschrieben.

2-Alkyl-2-Substituent-oxymethyl-1,3-diamino-propan-platin-Komplex-Derivate sind in EPA 0 263 956 beschrieben.

Platin-Komplexe, die eine Cyclobutan-1,1-dicarbonsäure als anionischer Ligand enthalten, besitzen meist hohe zytostatische Wirksamkeit im "in vivo" Test, müssen jedoch aufgrund der niedrigen Cytotoxizität im Vergleich zu den Dichlorid-Platin-Komplexen hoch dosiert werden.

Die vorliegende Erfindung hat sich zur Aufgabe gestellt, substituierte 1,3-Diaminopropan-N,N′-platin-Komplexe herzustellen, die bei niedrigerer Dosierung ihre therapeutische Wirkung behalten.

Überraschenderweise zeigte sich, daß die synthetischen, bisher unzugänglichen 2-Ethyl-2-methoxymethyl-1,3-diaminopropan-N,N′-platin-Komplexe, die Cyclopropan-1,1-dicarbonsäure oder Glycolsäure als Ligand enthalten, bei niedrigerer Dosis als die entsprechende Cyclobutan-1,1-dicarbonsäure-Derivate kurative antitumorale Wirkung besitzen.

Ausgehend von dieser Erkenntnis hatte es sich die vorliegende Erfindung zur Aufgabe gestellt, neue am C-2-Atom substituierte 1,3-Diamino-propan-N,N′-platin-Komplexe, die eine Dicarbonsäure oder alpha-Hydroxycarbonsäure als anionische Liganden enthalten, herzustellen, und deren Nützlichkeit als Zytostatika zu überprüfen.

Gelöst wurde diese Aufgabe durch die Verbindungen der Formel I.

Gegenstand der Erfindung sind Cis-platin-(II)-diamin-Komplexe der Formel I

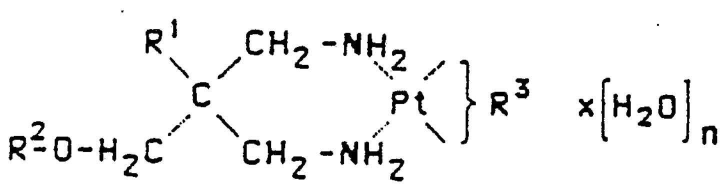

I

worin

$R^1$ $C_1$-$C_6$-Alkyl oder $C_1$-$C_4$-Alkyloxymethyl,

$R^2$ $C_1$-$C_4$-Alkyl oder $C_1$-$C_4$-Alkyloxyethyl,

$R^1$ und $R^2$ zusammen eine Ketal-bildende Gruppe -$CH_2$-O-$CR^4(R^5)$- mit

$R^4$ H oder $CH_3$ und

$R^5$ $CH_3$ oder Phenyl und

$R^3$ ein Anion der Cyclopropan-1,1-dicarbonsäure, Glycolsäure, alpha-$C_1$-$C_4$-Alkylglycolsäure, $C_1$-$C_4$-Alkyloxymalonsäure, Mesoxalsäure, Morpholin-4-yl-malonsäure oder Di-($C_1$-$C_4$-alkyl)-amino-malonsäure und

n 0, 1 oder 2 bedeuten.

Bevorzugt werden in Rahmen der Erfindung Verbindungen der allgemeinen Formel I, worin die Reste

$R^1$ Methyl, Ethyl oder Methoxymethyl,

$R^2$ Methyl oder Methyloxyethyl,

$R^1$ und $R^2$ zusammen eine Ketal-bildende Gruppe -$CH_2$-O-$CR^4(R^5)$- mit

$R^4$ H oder $CH_3$ und

$R^5$ $CH_3$ oder Phenyl,

$R^3$ ein Anion der Cyclopropan-1,1-dicarbonsäure, Glycolsäure, 2-Hydroxy-2-methyl-buttersäure, Methoxymalonsäure, Mesoxalsäure oder Morpholin-4-yl-malonsäure und
n 0,1 oder 2 bedeuten.

Die erfindungsgemäßen Verbindungen der Formel I sind dadurch herstellbar, daß man eine Verbindung der Formel II

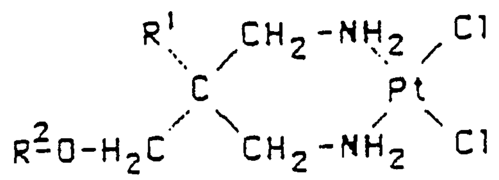

II

worin
$R^1$ $C_1$-$C_6$-Alkyl oder $C_1$-$C_4$-Alkyloxymethyl,
$R^2$ $C_1$-$C_4$-Alkyl oder $C_1$-$C_4$-Alkyloxyethyl,
$R^1$ und $R^2$ zusammen einer Ketal-bildenden Gruppe $-CH_2-O-CR^4(R^5)-$ mit
$R^4$ H oder $CH_3$ und
$R^5$ $CH_3$ oder Phenyl sind,
mit Cyclopropan-1,1-dicarbonsäure, Glycolsäure, alpha-($C_1$-$C_4$)-Alkylglycolsäure, $C_1$-$C_4$-Alkyloxymalonsäure, Mesoxalsäure, Morpholin-4-yl-malonsäure oder Di-($C_1$-$C_4$-alkyl)-amino-malonsäure bei Anwesenheit von Silberoxid oder einem Silbersalz dieser Säure in Wasser oder in Wasser unter Zusatz von 10 - 70 % eines mit Wasser mischbaren Lösungsmittels bei 0° bis 60° C unter Lichtausschluß umsetzt.

Die Ausgangsverbindungen der allgemeinen Formel II können nach den in EPA 0 174 542 und 0 263 956 beschriebenen Verfahren hergestellt werden.

Die Bestimmung der zytostatischen Wirksamkeit der neuen Verbindungen der Formel I erfolgte in vitro an L1210-Leukämiezellen der Maus sowie in vivo an L1210-Leukämie, B16-Melanoma und M 5076 Retikulumzellsarkom der Maus. Die akute Toxizität der Verbindungen wurde an BDF1-Mäusen ermittelt. Unter Berücksichtigung der geringen akuten Toxizität erweisen sich die erfindungsgemäßen Verbindungen dem Cisplatin überlegen hinsichtlich Löslichkeit und Wirksamkeit an der L1210-Leukämie in vivo. Des weiteren sind die erfindungsgemäßen Verbindungen auch bei Tumorzellen mit Resistenz gegen Cisplatin oder Carboplatin wirksam.

Gegenstand der Erfindung sind auch Arzneimittel, vorzugsweise zur Tumortherapie, die eine wirksame Menge einer oder mehrerer der Verbindungen der Formel I als Wirkstoff enthalten.

Die Dosierungs- und Anwendungsweise entspricht im wesentlichen der für Cisplatin oder Carboplatin bekannten, wobei aber aufgrund des günstigen therapeutischen Indexes der erfindungsgemäßen Verbindungen auch höhere Dosierungen und/oder eine häufigere Verabreichung in Frage kommen.

Neben den üblichen pharmazeutischen Konfektionierungs-und/oder Verdünnungsmitteln können diese Arzneimittel neben den Verbindungen der Formel I zur Unterstützung der Therapie ggf. auch noch weitere Wirkstoffe enthalten, sofern diese mit den erfindungsgemäßen Verbindungen der Formel I zusammen keine unerwünschten Nebenwirkungen zeigen.

## Beispiele

Die in den folgenden Beispielen beschriebenen Reaktionen sowie die Endprodukte wurden mittels HPLC (RP-18-Kieselgel, Elutionsmittel: Wasser/Methanol, UV-Detektion 210 nm) untersucht. Die Struktur der Verbindungen wurde mittels $^{13}C$-NMR-Spektroskopie und Elementaranalyse ermittelt.

In den folgenden Beispielen wird die vorliegende Erfindung näher beschrieben, ohne sie hierbei einzuschränken.

### Beispiel 1

Herstellung von Platin-Komplexen mit Cyclopropan-1,1-dicarbonsäure-Ligand

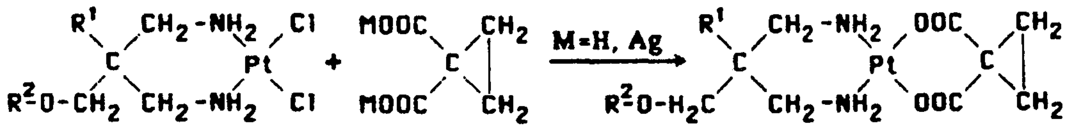

1,3-Diamino-2-ethyl-2-methoxymethyl-propan-N,N′-platin(II)-O,O′-cyclopropan-1,1-dicarboxylat (Verbindung 1)

2 g 1,3-Diamino-2-ethyl-2-methoxymethyl-propan-N,N′-platin(II)-dichlorid wurden in 50 ml Wasser/Methanol (1:1) suspendiert. Nach der Zugabe von 0.62 g Cyclopropan-1,1-dicarbonsäure und 1.1 g $Ag_2O$ wurde die Suspension 24 Stunden bei Raumtemperatur unter Lichtausschluß gerührt. Der Reaktionsansatz wurde abfiltriert, und das Filtrat wurde in Vakuum eingedampft. Der Rückstand wurde in wenig Methanol aufgenommen und mit Essigsäureethylester ausgefällt.
Ausbeute: 2 g (88 %), Schmelzpunkt: 222-224° C
$^{13}C$-NMR (90 MHz, $D_2O$, $\delta$) 180.04, 76.64, 58.89, 48.87, 40.15, 29.80, 24.87, 21.92, 6.28 ppm
(Interner Standard Dioxan).

1,3-Diamino-2-methoxymethyl-2-methyl-propan-N,N′-platin(II)-O,O′-cyclopropan-1,1-dicarboxylat (Verbindung 2)

1.5 g 1,3-Diamino-2-methoxy-methyl-2-methyl-propan-N,N′-platin(II)-dichorid und 1.29 g Silbersalz der 1,1-Cyclopropandicarbonsäure wurden in 30 ml Wasser/Methanol (1:1) 24 Stunden unter Lichtausschluß gerührt. Der Reaktionsansatz wurde, wie bei der Verbindung 1, aufgearbeitet.
Ausbeute: 1.5 g (87.6 %), Schmelzpunkt: 223-226° C

| Elementaranalyse: | | | | |
|---|---|---|---|---|
| Ber.: | C 27.91 % | H 4.68 % | N 5.92 % | Pt 41.21 % |
| Gef.: | C 27.72 % | H 4.68 % | N 5.90 % | Pt 40.87 % |

Die Titelverbindung enthält 1 mol $H_2O$.

1,3-Diamino-2-ethyl-2-(2′-methoxy-ethoxy-methyl)-propan-N,N′-platin(II)-O,O′-cyclopropan-1,1-dicarboxylat (Verbindung 3)

Die Titelverbindung wurde ausgehend von 2 g 1,3-Diamino-2-ethyl-2-(2′-methoxy-ethoxy-methyl)-propan-N,N′-platin(II)-dichlorid und 1.5 g Cyclopropan-1,1-dicarbonsäure Silbersalz, wie oben beschrieben, hergestellt.
Ausbeute: 1.75 g (77 %)
Elementaranalyse: Ber.: Pt 37.99 %, Gef.: 37.72 %

2,2-Bis-(methoxymethyl)-1,3-diamino-propan-N,N′-platin(II)-O,O′-cyclopropan-1,1-dicarboxylat (Verbindung 4)

Ausgehend von 2 g 2,2-Bis-(methoxymethyl)-1,3-diamino-propan-N,N′-platin(II)-dichlorid und 1.6 g Cyclopropan-1,1-dicarbonsäure Silbersalz wurde die Titelverbindung, wie oben beschrieben, hergestellt.
Ausbeute: 1.85 g (82.80 %)

| Elementaranalyse: | | | | |
|---|---|---|---|---|
| Ber.: | C 29.69 % | H 4.57 % | N 5.77 % | Pt 40.19 % |
| Gef.: | C 29.52 % | H 4.57 % | N 5.72 % | Pt 40.07 % |

5,5-Bis-(aminomethyl)-2,2-bis-(methyl)-1,3-dioxan-N,N'-platin(II)-O,O'-cyclopropan-1,1-dicarboxylat (Verbindung 5)

Ausgehend von 1 g 5,5-Bis-(aminomethyl)-2,2-bis-(methyl)-1,3-dioxan-N,N'-platin(II)-dichlorid und 0.78 g Cyclopropan-1,1-dicarbonsäure Silbersalz wurde die Titelverbindung, wie oben beschrieben, hergestellt.
Ausbeute: 0.95 g (85 %)
Elementaranalyse: Ber.: Pt 39.22 %, Gef.: 38.93 %

5,5-Bis-(aminomethyl)-2-phenyl-1,3-dioxan-N,N'-platin-(II)-O,O'-cyclopropan-1,1-dicarboxylat (Verbindung 6)

Ausgehend von 2 g 5,5-Bis-(aminomethyl)-2-phenyl-1,3-dioxan-N,N'-platin(II)-dichlorid und 1.41 g Cyclopropan-1,1-dicarbonsäure Silbersalz wurde die Titelverbindung, wie oben beschrieben, hergestellt.
Ausbeute: 1.95 g (87.2 %)

| Elementaranalyse: | | | | |
|---|---|---|---|---|
| Ber.: | C 37.43 % | H 4.07 % | N 5.14 % | Pt 35.77 % |
| Gef.: | C 37.27 % | H 4.06 % | N 5.07 % | Pt 35.61 % |

Beispiel 2

Herstellung von Platin-Komplexen mit 2-Methoxymalonsäure-Ligand

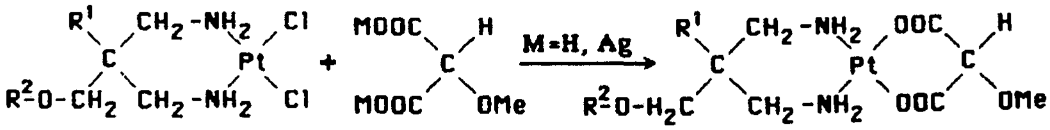


1,3-Diamino-2-ethyl-2-(2'-methoxy-ethoxy-methyl)-propan-N,N'-platin(II)-O,O'-2-methoxy-malonat (Verbindung 7)

1 g 1,3-Diamino-2-ethyl-2-(2'-methoxy-ethoxy-methyl)-propan-N,N'-platin(II)-dichlorid wurden in 40 ml Wasser/Methanol (2:1) suspendiert. Nach der Zugabe von 0.76 g 2-Methoxymalonsäure Silbersalz wurde der Reaktionsansatz 24 Stunden bei Raumtemperatur unter Lichtausshluß gerührt. Die Reaktionsmischung wurde abfiltriert, und das Filtrat wurde in Vakuum eingedampft. Der Rückstand wurde aus Methanol und Essigsäureethylester kristallisiert.
Ausbeute: 0.9 g (79 %)

| Elementaranalyse: | | | | |
|---|---|---|---|---|
| Ber.:<br>Gef.: | C 30.18 %<br>C 30.12 % | H 5.06 %<br>H 5.05 % | N 5.41 %<br>N 5.37 % | Pt 37.70 %<br>Pt 37.42 % |

1,3-Diamino-2,2-bis-(methoxymethyl)-propan-N,N′-platin(II)-O,O′-2-methoxymalonat (Verbindung 8)

1 g 1,3-Diamino-2,2-bis-(methoxymethyl)-propan-N,N′-platin(II)-dichlorid und 0.81 g 2-methoxy-malonsäure Silbersalz wurden zu der Titelverbindung, wie oben beschrieben, umgesetzt.
Ausbeute: 0.8 g (71 %)
Elementaranalyse: Ber.: Pt 39.86 %, Gef.: 39.83 %

1,3-Diamino-2-ethyl-2-methoxymethyl-propan-N,N′-platin(II)-O,O′-2-methoxymalonat (Verbindung 9)

Ausgehend von 1 g 1,3-Diamino-2-ethyl-2-methoxymethylpropan-N,N′-platin(II)-dichorid und 0.841 g 2-Methoxymalonsäure Silbersalz wurde die Titelverbindung, wie oben beschrieben, herstellt.
Ausbeute: 0.7 g (62 %), Schmelzpunkt: 213-215° C
Elementaranalyse: Ber.: Pt 41.21 %, Gef.: 41.08 %

1,3-Diamino-2-methoxymethyl-2-methyl-propan-N,N′-platin(II)-O,O′-2-methoxymalonat (Verbindung 10)

Ausgehend von 1 g 1,3-Diamino-2-methoxymethyl-2-methylpropan-N,N′-platin(II)-dichlorid und 870 mg 2-Methoxymalonsäure Silbersalz wurde die Titelverbindung, wie oben beschrieben, hergestellt.
Ausbeute: 0.8 g (69.6 %)
Elementaranalyse: Ber.: Pt 42.47 %, Gef.: 42.32 %

5,5-Bis-(aminomethyl)-2,2-bis-methyl-1,3-dioxan-N,N′-platin(II)-O,O′-2-methoxymalonat (Verbindung 11)

Ausgehend von 1 g 5,5-Bis-(aminomethyl)-2,2-bis-(methyl)-1,3-dioxan-N,N′-platin(II)-dichlorid und 787 mg 2-Methoxymalonsäure Silbersalz wurde die Titelverbindung, wie oben beschrieben, hergestellt.
Ausbeute: 1.0 g (90 %)

| Elementaranalyse: | | | | |
|---|---|---|---|---|
| Ber.:<br>Gef.: | C 28.75 %<br>C 28.62 % | H 4.42 %<br>H 4.42 % | N 5.59 %<br>N 5.58 % | Pt 38.91 %<br>Pt 38.73 % |

Beispiel 3

Herstellung von Platin-Komplexen mit 2-(4′-Morpholinyl)-malonsäure-Ligand

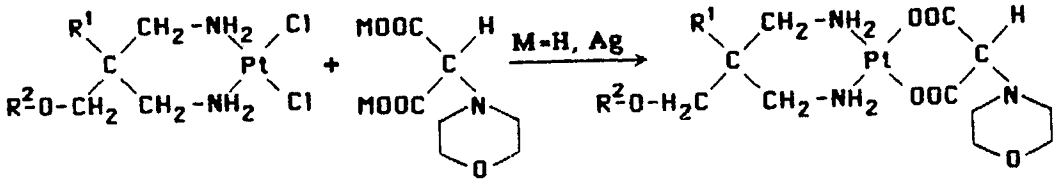


1,3-Diamino-2,2-bis-methoxymethyl-propan-N,N′-platin(II)-O,O′-2-(4′-morpholinyl)-malonat (Verbindung 12)

2 g 1,3-Diamino-2,2-bis-methoxymethyl-propan-N,N′-platin(II)-dichlorid wurden in 60 ml Wasser/Methanol(2:1) aufgenommen. Nach der Zugabe von 1.88 g 2-(4′-Morpholinyl)-malonsäure Silbersalz wurde die Suspension 24 Stunden bei Raumtemperatur unter Lichtausschluß gerührt. Das ausgefallene AgCl wurde abfiltriert, und das Filtrat wurde in Vakuum eingedampft. Der Rückstand wurde aus Ethanol und Essigsäureethylester kristallisiert.
Ausbeute: 2.1 g (82 %)

| Elementaranalyse: | | | | |
|---|---|---|---|---|
| Ber.:<br>Gef.: | C 30.88 %<br>C 30.73 % | H 5.00 %<br>H 5.01 % | N 7.72 %<br>N 7.74 % | Pt 35.83 %<br>Pt 35.81 % |

1,3-Diamino-2-ethyl-2-methoxymethyl-propan-N,N′-platin(II)-O,O′-2-(4′-morpholinyl)-malonat (Verbindung 13)

2 g 1,3-Diamino-2-ethyl-2-methoxymethyl-propan-N,N′-platin(II)-dichlorid und 1.73 g 2-(4′-Morpholinyl)-malonsäure Silbersalz wurden, wie oben beschrieben, zu der Titelverbindung umgesetzt.
Ausbeute: 2.17 g (85 %)

| Elementaranalyse: | | | | |
|---|---|---|---|---|
| Ber.:<br>Gef.: | C 31.82 %<br>C 31.73 % | H 5.15 %<br>H 5.16 % | N 7.95 %<br>N 7.87 % | Pt 36.92 %<br>Pt 36.47 % |

Beispiel 4

Herstellung von Platin-Komplexen mit 2-Dimethylaminomalonsäure-Ligand

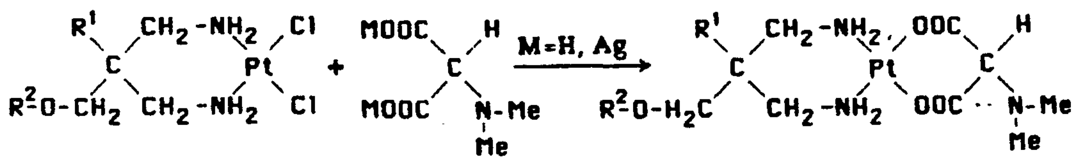


5,5-Bis-aminomethyl-2,2-bis-methyl-1,3-dioxan-N,N′-platin(II)-O,O′-2-dimethylamino-malonat (Verbindung 14)

2 g 5,5-Bis-aminomethyl-2-bis-methyl-1,3-dioxan-N,N′-platin(II)-dichlorid wurden in 60 ml Wasser/Methanol (2:1) suspendiert. Nach der Zugabe von 0.667 g 2-Dimethylamino-malonsäure und 1.05 g $Ag_2O$ wurde der Reaktionsansatz 24 Stunden bei Raumtemperatur unter Lichtausschluß gerührt. Das ausgefallene Silberchlorid wurde abfiltriert, und die Lösung wurde in Vakuum eingedampft. Der Rückstand wurd über RP-18 Kieselgelsäule (Elutionsmittel: Wasser/ Methanol) gereinigt.
Ausbeute: 1.93 g (83%)

| Elementaranalyse: | | | | |
|---|---|---|---|---|
| Ber.:<br>Gef.: | C 30.35 %<br>C 30.14 % | H 4.90 %<br>H 4.92 % | N 8.17 %<br>N 8.13 % | Pt 37.92 %<br>Pt 37.71 % |

1,3-Diamino-2-ethyl-2-methoxymethyl-propan-N,N′-platin(II)-O,O′-2-dimethylamino-malonat (Verbindung 15)

2 g 1,3-Diamino-2-ethyl-2-methoxymethyl-propan-N,N′-platin(II)-dichlorid, 0.713 g 2-Dimethylamino-malonsäure und 1.12 g $Ag_2O$ wurden, wie oben beschrieben, zu der Titelverbindung umgesetzt.
Ausbeute: 1.94 g (78 %)
Elementaranalyse:
Ber.: Pt 40.11 %, Gef.: 39.82 %

Beispiel 5

Herstellung von Platin-Komplexen mit Glycolsäure-Ligand

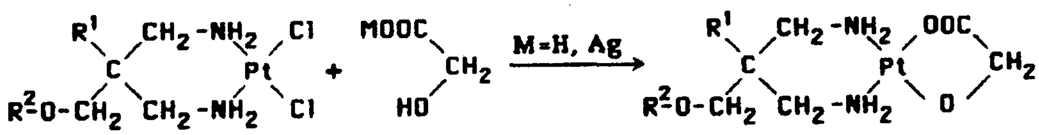


1,3-Diamino-2-ethyl-2-methoxymethyl-propan-N,N′-platin(II)-O,O′-2-hydroxy-acetat (Verbindung 16)

2 g 1,3-Diamino-2-ethyl-2-methoxymethyl-propan-N,N′-platin(II)-dichorid wurden in 60 ml Wasser/Methanol (2:1) suspendiert. Nach der Zugabe von 0.35 g Glycolsäure und 1.05 g $Ag_2O$ wurde der Reaktionsansatz 24 Stunden bei Raumtemperatur unter Lichtausschluß gerührt. Der Reaktionsansatz wurde abfiltriert, und das restliche feinverteilte AgCl wurde abzentrifugiert. Die verbleibende klare Lösung wurde in Vakuum eingedampft und der resultierende Rückstand in Methanol und Essigsäureethylester kristallisiert.
Ausbeute: 1.5 g (75.2 %), Schmelzpunkt: 185-187° C

| Elementaranalyse: | | | | |
|---|---|---|---|---|
| Ber.:<br>Gef.: | C 26.03 %<br>C 26.12 % | H 4.85 %<br>H 4.86 % | N 6.74 %<br>N 6.71 % | Pt 46.97 %<br>Pt 46.83 % |

1,3-Diamino-2-methoxymethyl-2-methyl-propan-N,N′-platin(II)-O,O′-2-hydroxy-acetat (Verbindung 17)

1 g 1,3-Diamino-2-methoxymethyl-2-methyl-propan-N,N′-platin(II)-dichlorid und 0.181 g Glycolsäure und 550 mg $Ag_2O$ wurden, wie oben beschrieben, zu der Titelverbindung umgesetzt.
Ausbeute: 900 mg (89.7 %), Schmelzpunkt: 300° C (Zersetzung)

| Elementaranalyse: | | | | |
|---|---|---|---|---|
| Ber.: | C 23.94 % | H 4.52 % | N 6.98 % | Pt 48.61 % |
| Gef.: | C 23.72 % | H 4.54 % | N 6.73 % | Pt 48.60 % |

$^{13}C$-NMR (90 MHz, $D_2O$,δ) : 194.24, 79.56, 67.94, 58.89, 51,26, 50.90, 37.41, 18.77 ppm

5,5-Bis-aminomethyl-2-phenyl-1,3-dioxan-N,N′-platin(II)-O,O′-2-hydroxy-acetat (Verbindung 18)

2 g 5,5-Bis-aminomethyl-2-phenyl-1,3-dioxan-N,N′-platin(II)-dichlorid, 296 mg Glycolsäure und 900 mg $Ag_2O$ wurden, wie oben beschrieben, zu der Titelverbindung umgesetzt.
Ausbeute: 1.8 g (82 %)
Elementaranalyse: Ber.: Pt 39.70 %, Gef.: 39.64 %

1,3-Diamino-2,2-bis-methoxymethyl-propan-N,N′-platin(II)-O,O′-2-hydroxy-acetat (Verbindung 19)

2 g 1,3-diamino-2,2-bis-methoxymethyl-propan-N,N′-platin(II)- dichlorid, 0.332 g Glycolsäure und 1 g $Ag_2O$ wurden, wie oben beschrieben, zu der Titelverbindung umgesetzt.
Ausbeute: 1.8 g (91 %)

| Elementaranalyse: | | | | |
|---|---|---|---|---|
| Ber.: | C 25.06 % | H 4.67 % | N 6.49 % | Pt 45.25 % |
| Gef.: | C 25.02 % | H 4.68 % | N 6.42 % | Pt 45.07 % |

1,3-Diamino-2-ethyl-2-(2′-methoxy-ethoxy-methyl)-propan-N,N′-platin(II)-O,O′-2-hydroxy-acetat (Verbindung 20)

2 g 1,3-Diamino-2-ethyl-2-(2′-methoxy-ethoxy-methyl)-propan-N,N′-platin(II)-dichlorid, 0.316 g Glycolsäure und 0.964 g $Ag_2O$ wurden, wie oben beschrieben, zu der Titelverbindung umgesetzt.
Ausbeute: 1.75 g (86.5 %)
Elementaranalyse: Ber.: Pt 42.46 %, Gef.: 41.97 %
$^{13}C$-NMR (90 MHz, $D_2O$,δ): 194.19, 77.26, 67.94, 58.87, 49.55, 49.14, 39.63, 25,25, 6.31 ppm

5,5-Bis-aminomethyl-2,2-bis-methyl-1,3-dioxan-N,N′-platin(II)-O,O′-2-hydroxy-acetat (Verbindung 21)

1 g 5,5-Bis-aminomethyl-2,2-bis-methyl-1,3-dioxan-N,N′-platin(II)-dichlorid, 0.164 g Glycolsäure und 0.5 g $Ag_2O$ wurden zu der Titelverbindung, wie oben beschrieben, umgesetzt.
Ausbeute: 0.90 g (89.4 %)
Elementaranalyse: Ber.: Pt 44.00 %, Gef.: 43.67 %

Beispiel 6

Herstellung von Platin-Komplexen mit 2-Hydroxy-2-methyl-buttersäure-Ligand

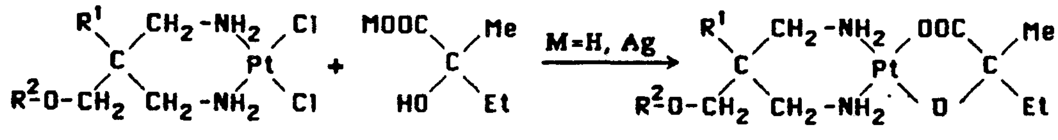


1,3-Diamino-2-ethyl-2-methoxymethyl-propan-N,N′-platin(II)-O,O′-(2-hydroxy-2-methyl)-butyrat (Verbindung 22)

1.5 g 1,3-Diamino-2-ethyl-2-methoxymethyl-propan-N,N′-platin(II)-dichlorid wurden in 120 ml Wasser/Methanol (1:1) suspendiert. Nach der Zugabe von 0.408 g 2-Hydroxy-2-methyl-buttersäure und 0.792 g $Ag_2O$ wurde der Reaktionsansatz 48 Stunden bei Raumtemperatur unter Lichtausschluß gerührt. Dann wurde das ausgefallene AgCl abfiltriert, und das Filtrat wurde in Vakuum eingedampft. Das resultierende Rohprodukt wurde säulenchromatographisch (Trägermaterial: Polystyrol XAD, Elutionsmittel: Wasser, Wasser/Methanol 4:1 und Wasser/Methanol 1:1) gereinigt.
Ausbeute: 1 g (60.7 %), Schmelzpunkt: 182-184° C
Elementaranalyse: Ber.: Pt 42.65 %, Gef.: 42.37 %
$^{13}$C-NMR (90 MHz, $D_2O$,δ): 197.52, 81.27, 77.31, 66.48 (Dioxan), 58.89, 49.63, 49.19, 39.60, 34.51, 27.25, 25.30, 7.91, 6.28 ppm
Interner Standard: Dioxan

5,5-Bis-aminomethyl-2,2-bis-methyl-1,3-dioxan-N,N′-platin(II)-O,O′-2-hydroxy-2-methyl)-butyrat (Verbindung 23)

1.5 g 5,5-Bis-aminomethyl-2,2-bis-methyl-1,3-dioxan-N,N′-platin(II)-dichlorid, 0.382 g 2-Hydroxy-2-methyl-buttersäure und 0.75 g $Ag_2O$ wurden zu der Titelverbindung, wie oben beschrieben, umgesetzt.
Ausbeute: 1.4 g (85 %)
Elementaranalyse: Ber.: Pt 40.19 %, Gef.: 40.03 %

Beispiel 7

Ermittlung der zytostatischen Aktivität

Die Bestimmung der zytostatischen Wirksamkeit der hier beschriebenen Verbindungen erfolgte an L1210-Leukämiezellen der Maus. Im einzelnen wurden folgende Testsysteme verwendet:

a) Koloniebildung von L1210-Leukämiezellen in soft agar

Diese Methode dient zum Nachweis eines Einflusses der Testsubstanzen auf das Wachstumsverhalten der Zellen über mehrere Generationen (bei einer Zellzykluszeit von 10 -12 Stunden werden in der Testzeit von 7 Tagen etwa 14 aufeinanderfolgende Generationen beobachtet). Zytostatisch wirksame Substanzen bewirken in diesem Test eine Reduktion der zu beobachtenden Koloniezahl gegenüber einer unbehandelten Kontrolle. Im einzelnen wird der Test wie folgt durchgeführt:
Leukämiezellen werden mit unterschiedlichen Konzentrationen von Testsubstanz 1 Stunde bei 37° C inkubiert.

Anschließend werden die Zellen zweimal mit McCoy5A-Medium gewaschen und schließlich in Petrischalen nach Zusatz von 0.3 % Agar ausgegossen (500 Zellen/Platte). Kontrollen werden lediglich mit frischem Medium inkubiert. Anstelle der einstündigen Inkubation werden in manchen Fällen unterschiedliche Konzentrationen und Testsubstanz der oberen Agarschicht zugemischt, um so eine kontinuierliche Exposition der Zellen über die gesamte Inkubationszeit zu erreichen. Nach Erstarren des Agars werden die Platten im Brutschrank 7 Tage bei 37° C inkubiert ( 5 Vol-% $CO_2$, 95 % relative Luftfeuch tigkeit). Anschließend wird die Anzahl der entstandenen Kolonien mit einem Durchmesser von mehr als 60 µ gezählt. Die

Ergebnisse werden angegeben als Koloniezahl in behandelten Agarplatten, in Prozent der unbehandelten Kontrolle. Aus der so erhalten Dosiswirkungskurve wird die $IC_{50}$ als Maß für die Wirksamkeit der Substanz ermittelt. Die Ergebnisse für die hier beschriebenen Verbindungen im Vergleich zu Cisplatin und Carboplatin wurden ermittelt.

b) Ermittlung der akuten Toxizität

Zur Ermittlung der akuten Toxizität werden NMRI-Mäusen am Tag 0 unterschiedliche Dosen der Testsubstanz gelöst in 0.5 ml 5 %iger Glucose-Lösung, intraperitoneal injiziert. Kontrollgruppen erhalten lediglich 0.5 ml 5 %ige Glucose-Lösung. Pro Konzentration der Testsubstanz werden 5 Mäuse verwendet. Am Tag 14 nach der letzten Injektion wird die Zahl der überlebenden Mäuse ermittelt und daraus nach der Lichtfield Wilcoxon Methode die LD5, LD50 und LD95 ermittelt. Die Toxizität (LD50 (mg/kg)) der hier beschriebenen Verbindungen im Vergleich zu Cisplatin und Carboplatin wurden ermittelt.

c) In vivo Wirksamkeit der Platin-Komplexe gegen L1210 Leukämie der Maus

Methodik:

Ascitesflüssigkeit wird unter sterilen Bedingungen DBA2 Mäusen (weiblich, 18 - 20 g) 7 Tage nach Implantation entnommen. Der Ascites wird dreimal mit PBS gewaschen, gezählt und auf eine Zellzahl von $10^6$ in 0.2 ml PBS eingestellt.
$10^6$ Zellen, suspendiert in 0.2 ml PBS, werden anschließend DBF1 Mäusen (weiblich, 18 - 20 g) intraperitoneal injiziert. 6 Tiere pro Gruppe werden für jede Substanz konzentration bzw. als Kontrolle eingesetzt.

Ermittlung der antitumoralen Wirksamkeit:

a) Die Tiere werden am Tag 1 und 5 nach Injektion der Testsubstanz gewogen. Gewichtsverlust von mehr als 20 % am Tag 5 wird als Indikator einer toxischen Substanzwirkung angesehen.

b) Am Ende des Experimentes (Tod aller Tiere oder überlebende Tiere am Tag 60) wird die mittlere Überlebenszeit der Tiere in den jeweiligen Gruppen bestimmt, sofern am Tag 5 des Experimentes mindestens 65 % der Tiere noch gelebt hatten.

Aus der mittleren Überlebenszeit ($MST_T$) der behandelten Gruppen sowie der Kontrollgruppen ($MST_c$) wird die antitumorale Wirksamkeit (T/C) für die jeweilige Substanzkonzentration in Prozent der unbehandelten Kontrolle entsprechend der folgenden Formel bestimmt:

$$T/C\ \% = \frac{MST_T}{MST_C} \times 100$$

T/C-Werte größer als 125 % werden als Anzeichen einer signifikanten antitumoralen Wirksamkeit der Testsubstanz angesehen. Die Dosis, die den größten antitumoralen Effekt (optimale Dosierung) sowie jeweils eine Dosisstufe oberhalb und unterhalb dieser Dosis wurden angegeben. Tiere, die am Tag 60 des Experimentes noch leben, werden als geheilt zusätzlich getrennt aufgeführt.

Die Daten der oben genannten Experimente sind in Tabelle 1 angegeben.

Tabelle 1:

| Verbindung Nr. | $IC_{50}$ (µg/ml) | $LD_{50}$ (mg/kg) 3 x i.p. | Behandlungsschema | T/C % (opt. Dose/LTS*) L1210 |
|---|---|---|---|---|
| 1 | 5.0 | 90-120 | 3 x i.p./i.p. | 450 % (50; 6/6) |
| 2 | 4.2 | | | |
| 9 | 2.35 | 60 | 3 x i.p./i.p. | 306 % (16; 3/6) |
| 16 | 1.2 | 30-60 | 3 x i.p./i.p. | 361 % (15.8; 4/6) |
| 17 | 1.8 | 30 | 3 x i.p./i.p. | 225 % (9.5; 1/6) |
| 22 | 2.9 | 90-180 | | |
| Cisplatin | 0.04 | 14 | 3 x i.p./i.p. | 157 % (4; -) |

LTS* = Long Term Survivor

## Ansprüche

1. Verbindung der Formel I

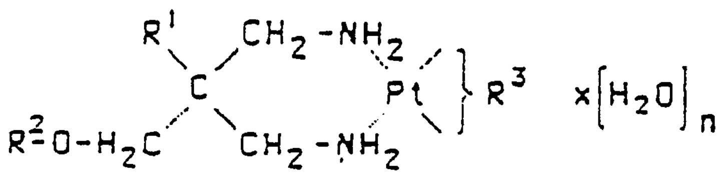

I

worin
$R^1$ $C_1$-$C_6$-Alkyl oder $C_1$-$C_4$-Alkyloxymethyl
$R^2$ $C_1$-$C_4$-Alkyl oder $C_1$-$C_4$-Alkyloxyethyl
$R^1$ und $R^2$ zusammen eine Ketal-bildende Gruppe $-CH_2-O-CR^4(R^5)-$ mit
$R^4$ H oder $CH_3$ und
$R^5$ $CH_3$ oder Phenyl und
$R^3$ ein Anion der Cyclopropan-1,1-dicarbonsäure, Glycolsäure, alpha-$C_1$-$C_4$-Alkylglycolsäure, $C_1$-$C_4$-Alkyloxymalonsäure, Mesoxalsäure, Morpholin-4-yl-malonsäure oder Di-($C_1$-$C_4$-alkyl)-amino-malonsäure und
n 0, 1 oder 2 bedeuten.

2. Verbindung nach Anspruch 1,
worin die Reste
$R^1$ Methyl, Ethyl oder Methoxymethyl,
$R^2$ Methyl oder Methyloxyethyl,
$R^1$ und $R^2$ zusammen eine Ketal-bildende Gruppe $-CH_2-O-CR^4(R^5)-$ mit
$R^4$ Wasserstoffatom oder Methyl und
$R^5$ Methyl oder Phenyl
$R^3$ ein Anion einer organischen Säure:
Cyclopropan-1,1-dicarbonsäure, Glycolsäure, 2-Hydroxy-2-methyl-buttersäure, Methoxymalonsäure, Mesoxalsäure oder Morpholin-4-yl-malonsäure und
n 0,1 oder 2 bedeuten.

3. Verbindung nach Anspruch 1,
worin die Reste
$R^1$ $C_1$-$C_6$-Alkyl,
$R^2$ $C_1$-$C_4$-Alkyl oder $C_1$-$C_4$-Alkyloxymethyl und
$R^3$ Cyclopropandicarboxylat und n = 0, 1 oder 2 sind.

4. Verbindung nach Anspruch 1,
worin die Reste

$R^1$ $C_1$-$C_4$-Alkyloxymethyl,
$R^2$ $C_1$-$C_4$-Alkyl oder $C_1$-$C_4$-Alkyloxyethyl
$R^1$ und $R^2$ zusammen eine Ketal-bildende Gruppe $-CH_2-OCR^4(R^5)-$ mit
$R^4$ H oder $CH_3$ und
$R^5$ $CH_3$ oder Phenyl
$R^3$ Cyclopropandicarboxylat und n = 0, 1 oder 2 sind.

5. Verbindung nach Anspruch 1,
worin die Reste $R^1$ und $R^2$ nach Anspruch 1 definiert sind und $R^3$ ein Anion der Glycolsäure ist.

6. Verbindung nach Anspruch 1,
worin die Reste $R^1$ und $R^2$ nach Anspruch 1 definiert sind und $R^3$ ein Anion der alpha-$C_1$-$C_4$-Alkylglycolsäure ist.

7. Verbindung nach Anspruch 1,
worin die Reste $R^1$ und $R^2$ nach Anspruch 1 definiert sind und $R^3$ ein Anion einer $C_1$-$C_4$-Alkyloxymalonsäure ist.

8. Verbindung nach Anspruch 1,
worin die Reste $R^1$ und $R^2$ nach Anspruch 1 definiert sind und $R^3$ ein Anion der Morpholin-4-yl-malonsäure ist.

9. Verbindung nach Anspruch 1,
worin die Reste $R^1$ und $R^2$ nach Anspruch 1 definiert sind und $R^3$ ein Anion einer Di-($C_1$-$C_4$)-alkylaminomalonsäure ist.

10. Verfahren zur Herstellung einer Verbinding nach Anspruch 1,
**dadurch gekennzeichnet,**
daß man eine Verbindung der Formel II

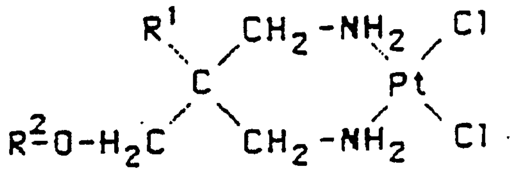

II

worin
$R^1$ $C_1$-$C_6$-Alkyl oder $C_1$-$C_4$-Alkyloxymethyl,
$R^2$ $C_1$-$C_4$-Alkyl oder $C_1$-$C_4$-Alkoxyethyl,
$R^1$ und $R^2$ zusammen eine Ketal-bildenden Gruppe $-CH_2-O-CR^4(R^5)-$ mit
$R^4$ H oder $CH_3$ und
$R^5$ $CH_3$ oder Phenyl sind,
mit Cyclopropan-1,1-dicarbonsäure, Glycolsäure, alpha-($C_1$-$C_4$)-Alkylglycolsäure, $C_1$-$C_4$-Alkyloxymalonsäure, Mesoxalsäure, Morpholin-4-yl-malonsäure oder Di-($C_1$-$C_4$-alkyl)-amino-malonsäure bei Anwesenheit von Silberoxid oder einem Silbersalz dieser Säure in Wasser oder in Wasser unter Zusatz von 10 - 70 % eines mit Wasser mischbaren Lösungsmittels bei 0° bis 60° C unter Lichtausschluß umsetzt.

11. Verbindung nach Anspruch 1 als Arzneimittel.

Patentansprüche für folgende Vertragsstaaten: ES, GR

1. Verfahren zur Herstellung einer Verbindung der Formel I

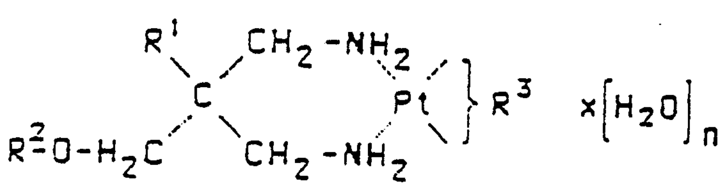

I

worin
$R^1$ $C_1$-$C_6$-Alkyl oder $C_1$-$C_4$-Alkyloxymethyl
$R^2$ $C_1$-$C_4$-Alkyl oder $C_1$-$C_4$-Alkyloxyethyl

$R^1$ und $R^2$ zusammen eine Ketal-bildende Gruppe $-CH_2-O-CR^4(R^5)-$ mit
$R^4$ H oder $CH_3$ und
$R^5$ $CH_3$ oder Phenyl und
$R^3$ ein Anion der Cyclopropan-1,1-dicarbonsäure, Glycolsäure, alpha-$C_1$-$C_4$-Alkylglycolsäure, $C_1$-$C_4$-Alkyloxymalonsäure, Mesoxalsäure, Morpholin-4-yl-malonsäure oder Di-($C_1$-$C_4$-alkyl)-amino-malonsäure und n 0, 1 oder 2 bedeuten,
**dadurch gekennzeichnet,**
daß man eine Verbindung der Formel II

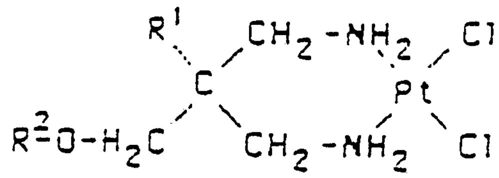

II

worin
$R^1$ $C_1$-$C_6$-Alkyl oder $C_1$-$C_4$-Alkyloxymethyl,
$R^2$ $C_1$-$C_4$-Alkyl oder $C_1$-$C_4$-Alkyloxyethyl,
$R^1$ und $R^2$ zusammen eine Ketal-bildende Gruppe $-CH_2-O-CR^4(R^5)-$ mit
$R^4$ H oder $CH_3$ und
$R^5$ $CH_3$ oder Phenyl sind,
mit Cyclopropan-1,1-dicarbonsäure, Glycolsäure, alpha-($C_1$-$C_4$)-Alkylglycolsäure, $C_1$-$C_4$-Alkyloxymalonsäure, Mesoxalsäure, Morpholin-4-yl-malonsäure oder Di-($C_1$-$C_4$-alkyl)-amino-malonsäure bei Anwesenheit von Silberoxid oder einem Silbersalz dieser Säure in Wasser oder in Wasser unter Zusatz von 10 -70 % eines mit Wasser mischbaren Lösungsmittels bei 0° bis 60° C unter Lichtausschluß umsetzt.

2. Verfahren nach Anspruch 1,
**dadurch gekennzeichnet,**
daß man eine Verbindung der Formel II, worin
$R^1$ Methyl, Ethyl oder Methoxymethyl und
$R^2$ Methyl oder Methyloxyethyl und die übrigen Definitionen wie in Anspruch 1 sind,
mit Cyclopropan-1,1-dicarbonsäure, Glycolsäure, 2-Hydroxy-2-methyl-buttersäure, Methoxymalonsäure, Mesoxalsäure oder Morpholin-4-yl-malonsäure unter den in Anspruch 1 angegebenen Bedingungen umsetzt.

Europäisches Patentamt

# EUROPÄISCHER RECHERCHENBERICHT

Nummer der Anmeldung

EP 89 11 8143

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A<br>D,X | EP-A-0 263 956 (BEHRINGWERKE AKTIENGESELLSCHAFT)<br>* Ansprüche 1,5; Seite 3, Zeile 26 - Seite 4, Zeile 12; Beispiel 4 *<br>--- | 1-10<br>11 | C 07 F 15/00<br>A 61 K 31/28 |
| D,A<br>D,X | EP-A-0 174 542 (BEHRINGWERKE AKTIENGESELLSCHAFT)<br>* Ansprüche 1-4; Seite 1, Zeile 1 - Seite 2, Zeile 16 *<br>--- | 1-10<br>11 | |
| A | EP-A-0 273 315 (SHI OGI SEIYAKU KABUSHIKA KAISHA)<br>* Ansprüche 1-3; Seite 2, Zeilen 23-43 *<br>--- | 1-6,11 | |
| A | EP-A-0 136 012 (KIDANI Y.)<br>* Ansprüche 1,2; Seite 1, Zeile 5 - Seite 2, Zeile 5 *<br>----- | 1,2,11 | |
| | | | RECHERCHIERTE SACHGEBIETE (Int. Cl.5) |
| | | | C 07 F 15/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 05-01-1990 | DAY G.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)